# EUROPEAN PATENT APPLICATION

(11) **EP 1 878 463 A1**
(43) Date of publication of application: **16.01.2008**
(21) Application number: 06014784.0
(22) Date of filing: 15.07.2006
(51) Int. Cl.: A61N 1/00

(54) **Music-synchronized low frequency stimulator**

(71) Applicant: Beaunix Co., Ltd., Cheonan-shi (KR)
(72) Inventor: Park, Jeong Hoon, Guangjin-gu, Seoul (KR)
(74) Representative: Dr. Weitzel & Partner

(57) **Abstract**

A music-synchronized low frequency stimulator is disclosed. In a low frequency stimulator that plays music such as classical music, pop song, folk song, etc. and generates a low frequency electric pulse in synchronization with the music for electrically stimulating and medically treating nerves of a human body, the music-synchronized low frequency stimulator comprises a sound output unit that outputs music such as classical music, pop song, folk song, etc. to a control unit; a control unit that outputs the music and simultaneously samples the music to generate a music-synchronized low frequency signal for stimulating various nerves of a human body; and a low frequency generation unit that generates a music-synchronized low frequency electric pulse in accordance with the control of the control unit.

## Description

### BACKGROUND OF THE INVENTION

### 1. Field of the Invention

The present invention relates to a music-synchronized low frequency stimulator, and in particular, to a music-synchronized low frequency stimulator that is capable of stimulating and medically curing the nerves of a certain portion of a human body by outputting music such as classical music, pop song, folk song, etc. and generating a low frequency electric pulse in synchronization with the outputted music.

### 2. Description of the Background Art

Generally, people listen to music and sing using a cassette tape recorder, a CD, a broadcast station of AM/FM, an audio device, an MP3 player, a sound output device such as Karaoke, etc. According to a study in psychoanalysis, a person feels the most peace and comfort in a mother's amniotic fluid pocket. In music therapy, it is known that water-based music largely influences human health. When a person is tired of studying or feels stress, listening to music such as Reflets dans l'eau by Claude Debussy, Jeux d'Eau by M. Ravel, water music by Handel, etc. or to record the sounds of water or nature and listen to the same is said to help provide rest and energy to the body and mind.

In addition, it is known that life activity or social life of a person has a certain cycle. Here, riding the above cycle is called "String" which is a technical term. When a person listens to a waltz, the person may feel a massage effect as dynamic rhythm is well matched with the tired mind and body. When a person listens to Sabre Dance by Aram Khachaturian, the person may feel increased strength in his body. So, anxiety, impatience, frustration, etc. that people living in the modern era feel may disappear by listening to calm, slow, dynamic or fast music, so that people may feel more comfort, rest and stability in mind.

In addition, a low frequency therapy device using a low frequency voltage pulse has been developed for enhancing blood circulation. The low frequency therapy device increases DC power to generate a low frequency signal around 100hz or less, and the above output is connected with conducting pads "+" and "-". The conducting pads are in direct contact with the person, so that the low frequency electric pulse can stimulate the human body.

The low frequency therapy device is placed at acupuncture spots of a human body or a portion where the person feels muscle pain to obtain a certain therapy effect.

In the low frequency therapy device, an output level may be adjusted in accordance with the control of a microcontroller that operates based on a method similar to volume level control.

The above output levels can be adjusted without set levels, but a digital control that utilizes microcontroller has not yet been obtained. Since the above output level is controlled based on an analog method, it is inconvenient to use. When the control and indication are performed using a microcontroller, it is possible to advantageously implement various controls, classified indications for expressing a control state and automated therapy courses which are performed in multiple stages.

According to the conventional art, in an output setting unit which sets a low frequency output of a microcontroller and variably controls the output, because different resistances are formed using potential divide resistors at four output ports of a microcontroller to adjust the potential divide ratio, it is possible to control the amplitude of an input power of a low frequency output unit. The output can be set to ten different levels that are a combined ratio of the potential divide resistors at four points.

The above low frequency therapy device is designed to stimulate the nerves using finger pressure protrusions or conductive pads that are attached to a foot sole or a portion of the human body. Because the conventional low frequency therapy device is designed to randomly generate low frequencies and stimulate the nerves of each portion of a human body, it is impossible to obtain a desired stimulation effect. When it is used for a long period of time, the user may feel discomfort, so that a desired therapy effect cannot be obtained.

### SUMMARY OF THE INVENTION

Accordingly, it is an object of the present invention to overcome the above-described problems that encountered in the conventional art.

It is another object of the present invention to provide a music-synchronized low frequency stimulator that is capable of stimulating and medically treating the nerves of a certain portion of a human body by generating a low frequency electric pulse that is synchronized with music such as classical music, pop song, folk song, etc.

It is further another object of the present invention to provide a music-synchronized low frequency stimulator that is capable of significantly enhancing the therapy effect by allowing a user to listen to music during the treatment.

To achieve the above objects, in a low frequency stimulator that plays classical music, pop song, folk song, etc. and generates a low frequency electric pulse in synchronization with the music to stimulate and medically treat various nerves of a human body, there is provided a music-synchronized low frequency stimulator that is comprised of a sound output unit that outputs music such as classical music, pop song, folk song, etc. and outputs the music to a control unit; a control unit that outputs the music and simultaneously samples the music and generates a music-synchronized low frequency signal for stimulating the nerves of a certain portion of a human body; and a low frequency generation unit that generates a music-synchronized low frequency electric pulse in accordance with the control of the control unit.

To achieve the above objects, in a low frequency stimulator that plays classical music, pop song, folk song, etc. and generates a low frequency electric pulse in synchronization with the generated music to stimulate and medically treat various nerves of a human body, there is provided a music-synchronized low frequency stimulator that is comprised of a sound output unit that includes an external input unit for receiving music from an external source, plays music such as classical music, pop song, folk song, etc. and provides the music to the control unit; a control unit that outputs the music and simultaneously samples the music and generates a music-synchronized low frequency signal to stimulate the nerves in a certain portion of a human body; and a low frequency generation unit that generates a music-synchronized low frequency electric pulse in accordance with the control of the control unit.

### BRIEF DESCRIPTION OF THE DRAWINGS

The present invention will become better understood with reference to the accompanying drawings that are given only by way of illustration, and thus, do not limit the present invention, wherein;
Figure 1 is a view illustrating a construction of a music-synchronized low frequency stimulator according to a preferred embodiment of the present invention;
Figure 2 is a view illustrating various low frequency therapy devices that adapt a music-synchronized low frequency stimulator according to the present invention;
Figure 3 is a view illustrating an inner block construction of a music-synchronized low frequency stimulator according to the present invention; and
Figure 4 is a view illustrating a construction of a music-synchronized low frequency stimulator according to another preferred embodiment of the present invention.

### DETAILED DESCRIPTION OF THE PREFERRED EMBODIMENTS

The music-synchronized low frequency stimulator, according to a preferred embodiment of the present invention, will be described with reference to the accompanying drawings.

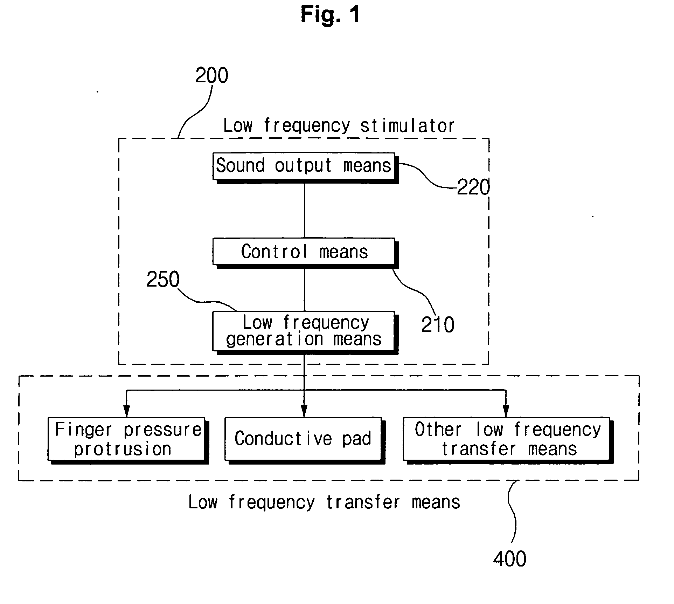

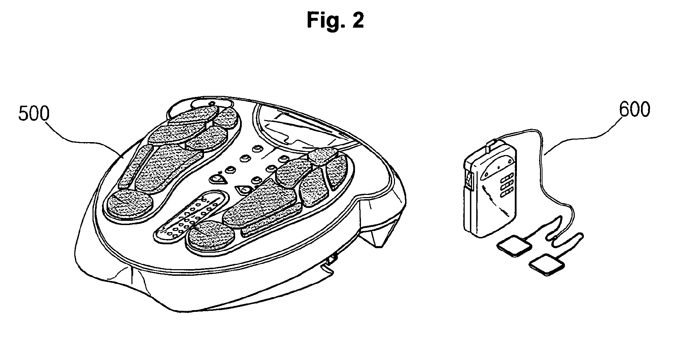

Figure 1 is a view illustrating a construction of a music-synchronized low frequency stimulator according to a preferred embodiment of the present invention, and Figure 2 is a view illustrating various low frequency therapy devices that adapt a music-synchronized low frequency stimulator according to the present invention. The low frequency stimulator will be described in more detail with reference to Figures 1 and 2.

The low frequency stimulator 200, according to the present invention, comprises a sound output unit 220, a control unit 210, and a low frequency generation unit 250.

The sound output device 220 is provided in the low frequency stimulator 200 and outputs music therein. The sound output device 200 directly processes analog music such as classical music, pop song, folk song, etc. that come from a cassette tape deck 222, a CD deck 224, an AM/FM tuner 226, a MP3 memory 228, etc. In addition, the sound output device 200 transfers the music to the control unit 210.

Here, the control unit 210 controls the sound output device 220 and manages music such as classical music, pop song, folk song, etc. from the cassette tape recorder deck 222, the CD deck 224, the AM/FM tuner 226, the MP3 memory 228, etc. In addition, the control unit 210 samples the reproduced music and converts the music into data that may be used for generating low frequencies in accordance with the volume level of the music and the output level of the low frequency signal so that the nerves of the human body can be stimulated.

The low frequency generation unit 250 generates low frequency electric pulse that is synchronized with the music from the sound output device 220 by dividing the intensity and speed of the low frequency signals into ten levels by using multiple potential divide resistors and multiple output level adjustments that control the input power. The low frequency generation unit 250 adjusts the speed and intensity of the low frequency electric pulse generated that is synchronized with the power and tempo of the sampled classical music, pop song, folk song, etc. The low frequency electric pulse is transferred to a user's body through finger pressure protrusions, conductive pads, or other low frequency transfer units, so that the nerves can be effectively stimulated.

As shown in Figure 2, the low frequency stimulator 200 comprises a foot reflection unit 500 that treats foot soles using pressure protrusions, a low frequency device 600 that treats various nerves by attaching the conductive pads to multiple portions of a human body, with the conductive pads being made of electric wires, and other low frequency generation units. There is further provided an external output unit 240 that includes output terminals connected to speakers, headphone, etc. for externally outputting music.

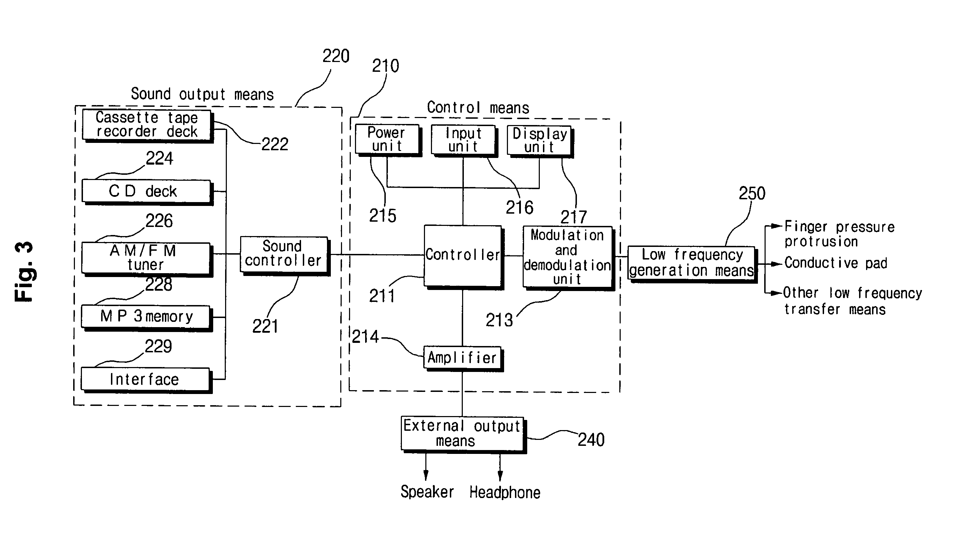

Figure 3 is a view illustrating an inner block construction of a music-synchronized low frequency stimulator according to the present invention. The music-synchronized low frequency stimulator according to the present invention will be described with reference to Figure 3.

The sound output unit 220 comprises a sound controller 221, a cassette tape recorder deck 222, a CD deck 224, an AM/FM tuner 226, an MP3 memory 228, and an interface 229.

The sound controller 221 outputs music such as classical music, pop song, folk song, etc. from the cassette tape recorder deck 222, the CD deck 224, the AM/FM tuner 226, or the MP3 memory 228 in accordance with the control of the control unit 210 and transfers the music to the control unit 210 by controlling the outputted music.

The cassette tape recorder deck 222 loads a common cassette tape and outputs classical music, pop song, folk song, etc. recorded on the cassette tape.

The CD deck 224 loads a common CD and outputs classical music, pop song, folk song, etc. recorded on the CD.

The AM/FM tuner 226 receives a broadcasted signal from the broadcast station and outputs the same.

The MP3 memory 228 is a detachable memory and downloads a music file through a wired network, wireless internet or an external memory or stores music files such as MP3 files, etc. The MP3 file stored by the MP3 memory 228 is outputted as music.

The interface 229 allows the music files of MP3 to be downloaded from a wired network, wireless internet or an external memory. Here, the interface 229 allows a download of an MP3 music file using a personal terminal or a terminal that can be connected to a wired network or wireless internet through a USB port, serial or parallel port, infrared ray port, etc.

Next, the control unit 210 comprises a controller 211, a modulation and demodulation unit 213, an amplifier 214, a power unit 215, an input unit 216, and a display unit 217.

The controller 211 processes music such as classical music, pop song, folk song, etc. from the sound output unit 220 and outputs the above music through an external speaker, a headphone, etc. using the amplifier 214 and the external output unit 240. In addition, the controller 211 analyzes music and extracts high and low frequencies sounds and tempo of music through a sampling process. The intensity and speed of the generated low frequency signal is synchronized with the high and low frequency sounds and tempo of the sampled music to generate the low frequency electric pulse.

The controller 211 allows the music data to be received through a wired network, wireless internet or from an external memory and to be stored in the MP3 memory 228.

The modulation and demodulation unit 213 analyzes analog or digital music such as classical music, pop song, folk song, etc. from the cassette tape recorder deck 222, the CD deck 224, the AM/FM tuner 226, or the MP3 memory 228 based on a sampling method. During the sampling performed by the modulation and demodulation unit 213, it is preferred to extract the high and low frequency sounds and tempo of music in synchronization with intensities and speed of the low frequency electric pulse generated by the low frequency generation unit 250.

The amplifier 214 amplifies analog or digital music such as classical music, pop song, folk music, etc. from the cassette tape recorder deck 222, the CD deck 224, the AM/FM tuner 226, or the MP3 memory 228 and externally outputs the music through the speaker and headphone using the external output unit 240.

The power unit 215 supplies power to the low frequency stimulator 200.

The input unit 216 provides selection keys formed of numbers and characters to allow the user to select various menu options such as music selection, high and low frequency sounds of music, tempo of music, low frequency generation intensity, and low frequency generation speed with respect to the music from the cassette tape recorder 222, the CD deck 224, the AM/FM tuner 226, the MP3 memory 228, etc.

The display unit 217 displays various menus for high and low frequency sounds of music, tempo of music, low frequency generation intensity, low frequency generation speed, and indication of low frequency from the cassette tape recorder 222, the CD deck 224, the AM/FM tuner 226, or the MP3 memory 228.

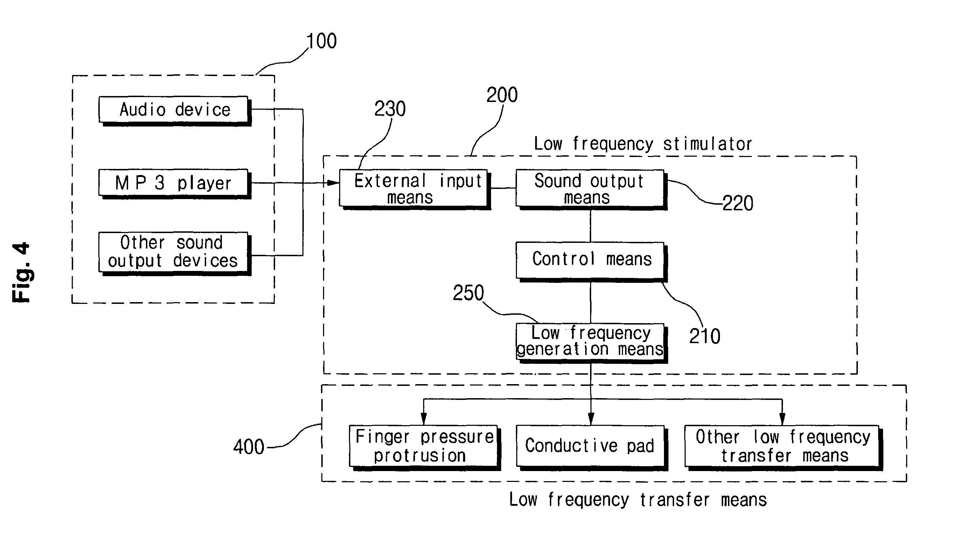

Figure 4 is a view illustrating a construction of a music-synchronized low frequency stimulator according to another preferred embodiment of the present invention. As shown therein, the low frequency stimulator 200 comprises a sound output unit 220 that has an external input unit 230, a control unit 210, and a low frequency generation unit 250 and receives music from an external sound output device 100 such as an audio device, an MP3 player, or other sound output devices to generate a music-synchronized low frequency electric pulse.

The sound output unit 220 is provided in the low frequency stimulator 200 to output music. It also includes an external input unit 230 that receives external music.

The sound output unit 220 outputs music by directly receiving music from the cassette tape recorder deck 222, the CD deck 224, the AM/FM tuner 226, or the MP3 memory 228 that are designed to output music such as classical music, pop song, folk song, etc. The sound output unit 220 outputs the sound to the control unit 210, which generates a music-synchronized low frequency signal.

The external input unit 230 includes an input terminal that receives various music such as classical music, pop song, folk song, etc. from an external audio device, an MP3 player, or other sound output devices.

The control unit 210 comprehensively manages music such as classical music, pop song, folk song, etc. from the cassette tape recorder 222, the CD deck 224, the AM/FM tuner 226, or the MP3 memory 228 in accordance with the control of the sound output unit 220 and generates a low frequency signal in accordance with the volume level of music and the output level of the low frequency signal by sampling the music and converting into data capable of outputting a low frequency for electrically stimulating the nerves of a human body.

The low frequency generation unit 250 generates a low frequency electric pulse in synchronization with music from the sound output unit 220 in accordance with the control of the control unit 210 that sets the intensity and speed of the low frequency signal at ten different levels by utilizing multiple potential divide resistors and multiple output adjustments that are, in turn, set by user input. The low frequency generation unit 250 outputs a low frequency electric pulse by adjusting the intensity and speed of the low frequency generation in synchronization with the high and low frequency sounds and tempo of the sampled classical music, pop song, folk song, etc. Here, the low frequency electric pulse is transferred to a certain portion of a human body through a finger pressure protrusion, a conductive pad, or other low frequency transfer units that electrically stimulate the nerves of a human body.

Because the constructions of the sound output unit 220, the control unit 210, and the low frequency generation unit 250 are the same as the previously described constructions with reference to Figure 3, the detailed description thereof will be omitted.

The operations of the music-synchronized low frequency stimulator according to the preferred embodiments of the present invention will be described.

First, the user turns on the low frequency stimulator 200 and selects a certain output level of the low frequency electric pulse and an output level of music on the menu displayed on the display unit 217 by using the input unit 216 of the control unit 210 and plays classical music, pop song, folk song, etc. through the sound output device 220 such as the cassette tape recorder deck 222, the CD deck 224, the AM/FM tuner 226, or the MP3 memory 228 and connects a headphone with the external output unit 230.

The music is managed and controlled by the sound controller 221 and is transmitted to the controller 211 of the control unit 210.

The amplifier 214 amplifies the music in accordance with the control of the controller 211 and outputs the music to an external output unit 240 through a headphone, so the user can listen to music such as classical music, pop song, folk song, etc.

The modulation and demodulation unit 213 analyzes the music in accordance with the control of the controller 211 by extracting and sampling the high and low frequency sounds and tempo of the music in synchronization to synchronize the intensity and speed of the generated low frequency signal. The low frequency electric pulse is generated in accordance to the intensity and speed depending on multiple potential divide resistors that are set by user input and that control the power from the low frequency generation unit 250 in synchronization with the sampled high and low frequency sounds and tempo of the music.

The generated low frequency electric pulse stimulates the user's body or a certain portion of the user's body through a finger pressure protrusion, a conductive pad, or other low frequency transfer units in synchronization with the high and low frequency sounds and tempo of the music.

As described above, in the music-synchronized low frequency stimulator according to the present invention, because the low frequency electric pulse is generated in synchronization with classical music, pop song, folk song, etc. selected by the user as compared to the low frequencies which are randomly generated by conventional art, the user can be provided with low frequency treatments while listening to music, and thereby maximizing the therapy effect.

As the present invention may be embodied in several forms without departing from the spirit or essential characteristics thereof, it should also be understood that the above-described examples are not limited by any of the details of the foregoing description, unless otherwise specified, but rather should be construed broadly within its spirit and scope as defined in the appended claims, and therefore all changes and modifications that fall within the meets and bounds of the claims, or equivalences of such meets and bounds are therefore intended to be embraced by the appended claims.

## Claims

1. In a low frequency stimulator that can play music such as classical music, pop song, folk song, etc. and generates a low frequency electric pulse in synchronization with the music to electrically stimulate and medically treat various nerves of a human body, a music-synchronized low frequency stimulator, comprising:
a sound output means that outputs music such as classical music, pop song, folk song, etc. to a control means;
a control means that outputs the music and simultaneously samples the music to generate a music-synchronized low frequency to stimulate various nerves of a human body; and
a low frequency generation means that generates a music-synchronized low frequency electric pulse in accordance with the control of the control means.

2. The system of claim 1, wherein said sound output means comprises:
a sound control unit that outputs sound from a cassette tape recorder deck, a CD deck, an AM/FM tuner, a MP3 memory, etc. and transfers the sound to the control means;
a cassette tape recorder deck that plays music recorded on a cassette tape;
a CD deck that plays music stored in a CD;
an AM/FM tuner that receives and outputs broadcasted signal from a broadcast station;
an MP3 memory that is detachable and downloads a music file such as an MP3 through a wired or wireless internet and stores the same; and
an interface that provides the function of downloading a music file such as an MP3 through a wired or wireless internet or an external memory.

3. The system of claim 1, wherein said control means comprises:
a control unit that controls the music from the sound output means, analyzes the music, extracts the high and low frequency sounds and tempo of the music through a sampling process, and generates a low frequency signal based on the intensity and speed selected by the user and synchronized to the high and low frequency sounds and tempo of the sampled music;
a power unit that supplies power to the low frequency stimulator;
an input unit that allows a user to select an output means such as a cassette tape recorder deck, a CD deck, an AM/FM tuner, or an MP3 memory and to select the desired high and low frequencies of sound, a low frequency generation intensity and a low frequency generation speed;
a display unit that displays various menus like selection of a sound output means such as a cassette tape recorder deck, a CD deck, an AM/FM tuner or an MP3 memory, high and low frequencies of sound, tempo of the music, a low frequency intensity and a low frequency generation speed for generating low frequency signals;
a modulation and demodulation unit that analyzes analog or digital music from the cassette tape recorder deck, the CD deck, the AM/FM tuner or the MP3 memory and extracts high and low sound frequencies and tempo of the music so that the low frequency generation means can generate a music-synchronized low frequency electric pulse in accordance with a user-controlled intensity and speed; and
an amplifier that amplifies music from the cassette tape recorder deck, the CD deck, the AM/FM tuner, or the MP3 player in accordance with the control of the controller and outputs through an external output means such as a speaker, a headphone, etc.

4. The system of claim 3, wherein said sampling is synchronized by extracting the high and low sound frequencies and the tempo of the music that will be used to set the low frequency of the electric pulse generated by the low frequency generation means in accordance to the user-controlled intensity and speed.

5. The system of claim 1, wherein said low frequency generation means is designed to adjust the intensity and speed of the generated low frequency electric pulse, each at ten different levels, in synchronization with the high and low sound frequencies and the tempo of the sampled music by utilizing multiple potential divide resistors that control the power and multiple output levels.

6. The system of claim 1, wherein said low frequency generation means provides various interfaces such as a finger pressure protrusion, a conductive pad and other low frequency transfer means and transfers a music-synchronized low frequency electric pulse to a user's body or a portion of the user's body for thereby electrically stimulating the nerves.

7. In a low frequency stimulator that plays music such as classical music, pop song, folk song, etc. and generates a low frequency electric pulse in synchronization with the music to electrically stimulate and medically treat various nerves of a human body, a music-synchronized low frequency stimulator, comprising:
a sound output means that includes an external input means for receiving external music, that outputs music such as classical music, pop song, folk song, etc. and that provides the music to the control means;
a control means that outputs the music and simultaneously samples the music to generate a music-synchronized low frequency signal for stimulating various nerves of a human body; and
a low frequency generation means that generates a music-synchronized low frequency electric pulse in accordance with the control of the control means.

8. The system of claim 7, wherein said external input means includes an input terminal that receives various music such as classical music, pop song, folk song, etc. from an external audio device, an MP3 player and other sound output devices.

9. The system of claim 7, wherein said sound output means comprises:
a sound control unit that outputs music from an external audio device, such as a cassette tape recorder deck, a CD deck, an AM/FM tuner, an MP3 player or other sound output devices, and transfers the music to the control means;
a cassette tape recorder deck that outputs music recorded on a cassette tape;
a CD deck that outputs music stored in a CD;
an AM/FM tuner that receives and outputs a broadcasted signal from a broadcast station;
a MP3 memory that is detachable and downloads a music file such as an MP3 through a wired or wireless internet and stores the same; and
an interface that provides the function of downloading a music file such as an MP3 through a wired or wireless internet or an external memory.
